# FASCICULE DE BREVET EUROPEEN

(11) **EP 0 860 411 B1**
(45) Date de publication et mention de la délivrance du brevet: **29.10.2003**
(21) Numéro de dépôt: 98400384.8
(22) Date de dépôt: 18.02.1998
(51) Int. Cl.: C07C 2/30

(54) **Composition catalyque améliorée pour la conversion de l'éthylène en oléfines alpha légères**
Verbesserte katalytische Zusammensetzung zur Umwandlung von Ethylen in alpha-Olefine
Improved catalytic composition for the conversion of ethylene to alpha olefins

(30) Priorité: 25.02.1997 FR 9702328
(43) Date de publication de la demande: 26.08.1998
(73) Titulaire: Institut Français du Pétrole, 92500 Rueil Malmaison (FR)
(72) Inventeur: Commereuc, Dominique, 92190 Meudon (FR); Boivineau, Serg, 69600 Oullins (FR); Hugues, François, Charly 69390 Vernaison (FR); Saussine, Lucien, 78290 Croissy Sur Seine (FR)

(56) Documents cités:
- EP-A- 0 295 960
- EP-A- 0 320 571
- EP-A- 0 444 505
- WO-A-95/19332
- DE-C- 4 338 414
- US-A- 5 345 023

## Description

L'objet de la présente invention est une composition catalytique améliorée utilisable pour la production d'oléfines alpha légères par oligomérisation de l'éthylène. Ladite composition catalytique améliorée de type Ziegler est obtenue par mélange d'un composé de zirconium avec un composé organique choisi dans la classe des acétals et des cétals, avec un composé d'aluminium hydrocarbyl choisi dans la classe des composés chlorés ou bromés d'aluminium hydrocarbyl et avec un composé d'aluminium hydrocarbyl choisi dans la classe des tris(hydrocarbyl)-aluminium.

Un autre objet de l'invention est un procédé pour la production d'oléfines alpha légères par oligomérisation de l'éthylène.

De nombreux composés de zirconium ont été mis en oeuvre pour réaliser l'oligomérisation de l'éthylène en oléfines alpha, souvent associés à des coordinats variés.

On peut citer par exemple l'utilisation d'halogénures de zirconium associés à des esters, cétones, éthers, amines, nitriles, anhydrides, chlorures d'acides, amides ou aldéhydes, décrite dans les brevets US 4 855 525 et WO 91 02707, ou l'utilisation des mêmes halogénures de zirconium associés à des ligands sélectionnés parmi les groupes des composés soufrés, phosphorés ou azotés, décrite dans les brevets EP-A-241 596 et EP-A-328 728.

Les produits obtenus avec les formules catalytiques ci-dessus sont constitués principalement d'oléfines alpha ayant une longueur de chaîne située entre C₁₀ et C₁₈.

Le brevet EP-A-0 444 505 met en oeuvre un catalyseur de type Ziegler contenant ZXₐA₄₋ₐ, Al₂R₃Q₃ et/ou AlR¹_{b}Q¹_{3-b} et un troisième composant éventuel à base de soufre, phosphore, azote. Le problème traité dans EP-A-0 444 505 est l'élimination des cendres contenues dans le milieu réactionnel après oligomérisation des alpha-oléfines par un catalyseur de type Ziegler.

Dans le brevet US-A-5 345 023 est décrit un procédé de production d'oléfines alpha légères, principalement le butène-1, l'hexène-1, l'octène-1 et le décéne-1, par oligomérisation de l'éthylène, mettant en oeuvre une composition catalytique obtenue par mélange d'un composé de zirconium avec un composé organique choisi dans la classe des acétals et des cétals et avec un composé chloré ou bromé d'aluminium hydrocarbyl. Les catalyseurs sont obtenus en mélangeant un composé de zirconium avec au moins un composé organique choisi parmi la classe des acétals d'aldéhydes et des cétals de cétone, et avec au moins un composé particulier d'aluminium, présentent une sélectivité inattendue pour la formation des oligomères inférieurs, principalement le butène-1, l'hexène-1, l'octène-1 et le décéne-1, qui trouvent une utilisation comme comonomères avec l'éthylène dans la fabrication du polyéthylène basse densité linéaire, ou comme base de départ pour des huiles lubrifiantes de synthèse. Outre l'amélioration de la sélectivité pour les alpha-oléfines légères, les catalyseurs décrits ont également pour but de réduire à une quantité très faible le polymère sous-produit.

II a maintenant été trouvé que l'addition d'un tris-(hydrocarbyl)-aluminium dans cette composition catalytique permet d'en augmenter l'activité de façon très importante.

Plus précisément, ladite composition catalytique améliorée est obtenue par mélange :
- d'au moins un composé de zirconium de formule ZrXₓY_{y}O_{z} dans lequel X est un atome de chore ou de brome, Y est un radical choisi dans le groupe formé par les alkoxy RO⁻, les amido R₂N⁻, les carboxylates RCOO⁻, où R est un radical hydrocarbyl comprenant de 1 à 30 atomes de carbone, x et y peuvent prendre les valeurs entières de 0 à 4 et z est égal à 0 ou 0,5, la somme x + y + 2z étant égale à 4,
- avec au moins un composé organique de formule (R₁')(R₂')C(OR₁)(OR₂) dans laquelle R₁' et R₂' sont constitués par un atome d'hydrogène ou un radical hydrocarbyl comprenant de 1 à 30 atomes de carbone, R₁ et R₂ sont des radicaux hydrocarbyl comprenant de 1 à 30 atomes de carbone,
- avec au moins un composé d'aluminium de formule AlR"ₙX₃₋ₙ dans laquelle R" est un radical hydrocarbyl comprenant de 1 à 6 atomes de carbone, X est un atome de chlore ou de brome, et n est un nombre compris entre 1 et 2,
- et avec au moins un composé d'aluminium de formule AIR"'3 dans laquelle R"' est un radical hydrocarbyl comprenant de 1 à 6 atomes de carbone.

La composition comprend également de préférence au moins un solvant avantageusement choisi dans le groupe formé par les hydrocarbures aliphatiques, cycloaliphatiques et aromatiques, et les composés correspondant aux sous-produits d'oligomérisation tels que les oligomères supérieurs. De préférence, le solvant choisi parmi les hydrocarbures aromatiques, et l'orthoxylène convient particulièrement bien.

Pour les composés de zirconium, on peut citer à titre d'exemples les halogénures de zirconium tels que le tétrachlorure de zirconium ZrCl₄, le tétrabromure de zirconium ZrBr4, les alcoolates tels que le tétrapropylate de zirconium Zr(OC₃H₇)₄, le tétrabutylate de zirconium Zr(OC₄H₉)₄, les carboxylates tels que le tétra-éthyl-2-hexanoate de zirconium Zr(OCOC₇H₁₅)₄ ou les oxo-carboxylates comme l'oxo-hexaéthyl-2-hexanoate de dizirconium [Zr(OCOC₇H₁₅)₃]₂O.

Les composés organiques choisis parmi la classe des acétals et des cétals utilisés selon l'invention résultent de la condensation d'un aldéhyde ou d'une cétone avec un monoalcool ou un polyalcool, par exemple un glycol. Ils répondent à la formule générale suivante :

(R₁')(R₂')C(OR₁)(OR₂)

dans laquelle R₁' et R₂' sont constitués par un atome d'hydrogène ou un radical hydrocarbyl comprenant de 1 à 30 atomes de carbone, et R₁ et R₂ sont des radicaux hydrocarbyl comprenant de 1 à 30 atomes de carbone. Les deux radicaux R₁' et R₂' et les deux radicaux R₁ et R₂ peuvent être identiques ou différents. Ils peuvent également faire partie d'un cycle. On peut citer à titre d'exemples le diéthoxyméthane, le diisopropoxyméthane, le diéthoxy-1,1-éthane, le diisobutoxy-1,1-éthane, le diméthoxy-1,1-décane, le nonyl-2-dioxolane-1,3, le diméthoxy-2,2-octane, le diméthoxy-1,1-cyclohexane, et de préférence le diméthoxy-2,2-propane, le dibutoxy-2,2-propane, le dihexyloxy-2,2-propane, le dioctoxy-2,2-propane, le di-(éthyl-2-hexyloxy)-2,2-propane.

Pour les composés de l'aluminium utilisés dans l'invention représentés par la formule générale AlR"ₙX₃₋ₙ, on peut citer à titre d'exemples le chlorodiéthylaluminium, le dichloroéthylaluminium, et de préférence le sesquichlorure d'éthylaluminium, ou leurs mélanges.

Pour les composés de l'aluminium utilisés dans l'invention représentés par la formule générale AlR"'₃, on peut citer à titre d'exemples le triméthylaluminium, le tributylaluminium, le trihexylaluminium, et de préférence le triéthylaluminium.

De façon particulièrement avantageuse, la solution de catalyseur résulte de l'interaction d'un mélange d'au moins un composé de zirconium, tel que par exemple le tétrachlorure de zirconium, et d'au moins un composé organique choisi dans la classe des acétals et des cétals, résultant de la condensation d'un aldéhyde ou d'une cétone avec un monoalcool ou un polyalcool, tel que par exemple le di-(éthyl-2-hexyloxy)-2,2-propane, avec au moins un composé chloré ou bromé d'aluminium hydrocarbyl, tel que par exemple le sesquichlorure d'éthylaluminium, et avec au moins un composé tris-(hydrocarbyl)-aluminium, tel que par exemple le triéthylaluminium.

Les composants du catalyseur peuvent être mis en contact dans un ordre quelconque dans un solvant choisi dans le groupe formé par les hydrocarbures aliphatiques et cycloaliphatiques tels que l'hexane, le cyclohexane ou l'heptane, les hydrocarbures aromatiques tels que le toluène ou les xylènes, et les sous-produits d'oligomérisation tels que les oligomères supérieurs. On utilise avantageusement les hydrocarbures aromatiques, et de préférence l'ortho-xylène. De préférence, le composé de zirconium est d'abord mélangé avec l'acétal ou le cétal, puis les composés d'aluminium sont ajoutés au mélange, dans un ordre indifférent ou eux-mêmes en mélange.

Le rapport molaire entre l'acétal ou le cétal et le composé de zirconium est de 0,1:1 à 5:1, et de préférence de 0,5:1 à 2:1. Le rapport molaire entre le composé chloré ou bromé d'aluminium hydrocarbyl et le composé de zirconium est de 1:1 à 100:1, de préférence de 5:1 à 50:1. Le rapport molaire entre le composé de tris-(hydrocarbyl)-aluminium et le composé de zirconium est de 0,01:1 à 10:1, de préférence de 0,1:1 à 2:1. La concentration de zirconium dans la solution catalytique ainsi préparée est avantageusement comprise entre 10⁻⁴ et 1 mole par litre, et de préférence entre 10⁻³ et 0,5 mole par litre. La température à laquelle les quatre composants sont mélangés est normalement comprise entre -10 et +180 °C, de préférence entre 0 et +150 °C, par exemple à une température voisine de l'ambiante (15 à 30 °C). Le mélange peut être effectué sous une atmosphère d'éthylène ou de gaz inerte.

La composition selon l'invention est notamment utilisable dans les procédés de conversion de l'éthylène en oléfines alpha légères.

La solution ou la composition catalytique obtenue précédemment peut être utilisée telle quelle pour la mise en oeuvre de la réaction d'oligomérisation, ou elle peut être diluée par addition d'un solvant choisi dans le groupe formé par les hydrocarbures aliphatiques, cycloaliphatiques et aromatiques, et les sous-produits d'oligomérisation tels que les oligomères supérieurs. On utilise avantageusement les hydrocarbures aromatiques, et de préférence l'ortho-xylène.

Le procédé opère dans les conditions d'oligomérisation, avec des pressions de 0,5 à 15 MPa, des températures de 20-180 °C.

Dans un procédé de mise en oeuvre de la réaction catalytique d'oligomérisation en discontinu, on introduit un volume choisi de la solution catalytique, préparée comme décrit ci-dessus, dans un réacteur muni des habituels systèmes d'agitation et de refroidissement, puis on pressurise par de l'éthylène à une pression généralement comprise entre 0,5 et 15 MPa, de préférence entre 1 et 10 MPa, et on maintient la température en général entre 20 et 180 °C, de préférence entre 40 et 150 °C. On alimente le réacteur d'oligomérisation par de l'éthylène à pression constante jusqu'à ce que le volume total de liquide produit représente entre 2 et 50 fois le volume de la solution catalytique primitivement introduit. On détruit alors le catalyseur, par exemple par injection d'une amine dans le réacteur, puis on soutire et sépare les produits de la réaction et le solvant éventuel.

Dans un procédé opérant en continu, la mise en oeuvre est de préférence la suivante : la solution catalytique est injectée en même temps que l'éthylène dans un réacteur agité par les moyens mécaniques classiques ou par une recirculation extérieure. On peut aussi injecter séparément les composants du catalyseur dans le milieu réactionnel, par exemple le produit d'interaction du composé de zirconium avec l'acétal ou le cétal d'une part, et le mélange des deux composés d'aluminium d'autre part. La température est maintenue entre 20 et 180 °C, de préférence entre 40 et 150 °C, et la pression est ajustée généralement entre 0,5 et 15 MPa et de préférence entre 1 et 10 MPa. Le niveau liquide dans le réacteur est maintenu constant. L'éthylène est introduit par une vanne d'admission, asservie à la pression, qui maintient celle-ci constante. Le mélange réactionnel est soutiré de la zone réactionnelle au moyen d'une vanne asservie au niveau liquide. Ce mélange est envoyé dans une zone de destruction du catalyseur qui comporte par exemple d'abord l'injection d'une amine, puis une vaporisation de l'effluent traité par l'amine, soit par élévation de température, soit par abaissement de la pression, soit par action simultanée sur la température et la pression, de façon à recueillir les produits dans la fraction vaporisée. Les produits et le solvant éventuel sont ensuite séparés dans un système de colonnes à distiller. L'éthylène qui n'a pas réagi peut être renvoyé au réacteur. Les résidus de catalyseur inclus dans une fraction lourde peuvent être incinérés.

Les exemples suivants illustrent l'invention sans en limiter la portée.

### EXEMPLE 1

Dans un ballon en verre de 100 ml placé sous atmosphère inerte, on transfère à l'abri de l'humidité 2.10⁻³ mole de tétrachlorure de zirconium sublimé, puis on injecte au moyen d'une seringue hypodermique 45 ml d'ortho-xylène séché et désaéré. A la suspension blanche agitée à température ambiante au moyen d'un barreau magnétique, on ajoute 2.10⁻³ mole de di-(éthyl-2-hexyloxy)-2,2-propane en solution dans 5 ml d'ortho-xylène. En quelques minutes, le chlorure de zirconium se dissout et il se forme une solution homogène de couleur jaune.

Dans un autoclave en acier inoxydable d'un volume utile de 250 ml, muni d'une double enveloppe permettant de réguler la température par circulation d'eau ou d'huile, on introduit, dans l'ordre, sous atmosphère d'argon, et à la température ambiante, 5 ml de la solution de complexe de zirconium préparée ci-dessus, soit 0,2.10⁻³ mole de zirconium, 50 ml d'ortho-xylène, puis un mélange de 0,2.10⁻³ mole de triéthylaluminium et de 1,2.10⁻³ mole de sesquichlorure d'éthylaluminium Al₂Et₃Cl₃ en solution dans 10 ml d'ortho-xylène. La température est alors portée à 90 °C tout en introduisant dans l'autoclave de l'éthylène de manière à maintenir une pression constante de 6 MPa.

Après 2 heures de réaction, l'introduction d'éthylène est arrêtée. On injecte alors dans l'autoclave sous pression 5.10⁻³ mole d'éthyl-2-hexylamine en solution dans 3 ml d'ortho-xylène, au moyen d'un sas que l'on peut porter à une pression supérieure à celle de l'autoclave. L'autoclave est ensuite dépressurisé et on recueille une fraction gazeuse et une fraction liquide qui sont analysées par chromatographie.

Le bilan matière de la réaction montre qu'il s'est formé 92,3 g d'oligomères, ce qui correspond à une activité spécifique égale à 10140 g d'oligomères / g de zirconium / heure. La composition des oligomères est la suivante :

| | | | |
|---|---|---|---|
| butènes | 25,2 % en poids | butène-1 | 98,8 % |
| hexènes | 22,6 | hexène-1 | 96,5 |
| octènes | 16,9 | octène-1 | 94,0 |
| décènes | 12,9 | décène-1 | 86,6 |
| lourds | 22,4 | | |

### EXEMPLE 2 (Comparatif)

On utilise dans cet exemple le même mode opératoire que dans l'exemple 1 pour préparer la solution de complexe de zirconium. Le catalyseur est mis en oeuvre dans le même autoclave, mais on omet d'ajouter le triéthylaluminium et le seul composé d'aluminium utilisé est donc le sesquichlorure d'éthylaluminium avec la même quantité et la même concentration que dans l'exemple 1.

La réaction d'oligomérisation de l'éthylène est effectuée dans les mêmes conditions que dans l'exemple 1 et pendant le même temps de réaction. A la fin de la réaction, on injecte dans l'autoclave de l'éthyl-2-hexylamine dans les mêmes quantités et avec la même technique que dans l'exemple 1.

Le bilan matière de la réaction montre qu'il s'est formé 59 g d'oligomères, ce qui correspond à une activité spécifique égale à 6486 g d'oligomères / g de zirconium / heure. La composition des oligomères est la suivante :

| | | | |
|---|---|---|---|
| butènes | 25,5 % en poids | butène-1 | 96,2 % |
| hexènes | 24,1 | hexène-1 | 95,8 |
| octènes | 17,7 | octène-1 | 93,9 |
| décènes | 13 | décène-1 | 85,6 |
| lourds | 19,7 | | |

Par comparaison avec l'exemple 1 selon l'invention, cet exemple de l'art antérieur montre la nette supériorité de l'invention.

### EXEMPLE 3

La réaction est réalisée ici dans une unité pilote fonctionnant en mode continu. Celle-ci comprend un réacteur parfaitement agité d'un volume total de 3 litres, opérant avec un contrôle de niveau liquide à 2 litres. Dans ce réacteur, dont la température est régulée à 135°C au moyen d'une circulation d'huile et la pression maintenue à 8,5 Mpa grâce à une vanne située sur la ligne d'admission de l'éthylène, on injecte en continu 17,2 g/h d'une solution de 1 kg d'ortho-xylène contenant 0,37 g de chlorure de zirconium sublimé et 0,45 g de di-(éthyl-2-hexyloxy)-2,2-propane, et 17,2 g/h d'une solution de 1 kg d'ortho-xylène contenant 5,9 g de sesquichlorure d'aluminium et 0,19 g de triéthylaluminium, ainsi que 493 g/h d'ortho-xylène.

Dans ces conditions, le débit d'éthylène à l'entrée du réacteur, asservi à la pression, s'établit à 259 g/h. La production d'oligomères est de 136 g/h, ce qui correspond à une productivité de 54,5 kg/g Zr. La composition des oligomères est la suivante :

| | | | |
|---|---|---|---|
| butènes | 28,6 % en poids | butène-1 | 99,7 % |
| hexènes | 25,0 | hexène-1 | 98,1 |
| octènes | 18,3 | octène-1 | 96,4 |
| décènes | 12,2 | décène-1 | 93,0 |
| lourds | 15,9 | | |

### EXEMPLE 4 (Comparatif)

La réaction est effectuée dans le même appareillage et selon le même mode que décrit dans l'exemple précédent. Dans ce réacteur, dont la température est régulée à 135°C au moyen d'une circulation d'huile et la pression maintenue à 8,5 Mpa grâce à une vanne située sur la ligne d'admission de l'éthylène, on injecte en continu 26 g/h d'une solution de 1 kg d'ortho-xylène contenant 0,38 g de chlorure de zirconium sublimé et 0,46 g de di-(éthyl-2-hexyloxy)-2,2-propane, et 26 g/h d'une solution de 1 kg d'ortho-xylène contenant 5,9 g de sesquichlorure d'aluminium, ainsi que 508 g/h d'ortho-xylène.

Dans ces conditions, le débit d'éthylène à l'entrée du réacteur, asservi à la pression, s'établit à 273 g/h. La production d'oligomères est de 130 g/h, ce qui correspond à une productivité de 34,1 kg/g Zr. La composition des oligomères est la suivante :

| | | | |
|---|---|---|---|
| butènes | 28,8 % en poids | butène-1 | 99,5 % |
| hexènes | 26,4 | hexène-1 | 97,7 |
| octènes | 18,6 | octène-1 | 95,8 |
| décènes | 11,6 | décène-1 | 90,6 |
| lourds | 14,6 | | |

Par comparaison avec l'exemple 3, cet exemple démontre clairement l'avantage du catalyseur selon l'invention.

## Revendications

1. Composition catalytique, **caractérisée en ce que** ladite composition catalytique est obtenue par mélange :
- d'au moins un composé de zirconium de formule ZrXₓY_{y}O_{z} dans lequel X est un atome de chlore ou de brome, Y est un radical choisi dans le groupe formé par les alkoxy RO-, les amido R₂N-, les carboxylates RCOO-, où R est un radical hydrocarbyl comprenant de 1 à 30 atomes de carbone, x et y peuvent prendre les valeurs entières de 0 à 4 et z est égal à 0 ou 0,5, la somme x + y + 2z étant égale à 4,
- avec au moins un composé organique de formule (R₁')(R₂')C(OR₁) (OR₂) dans laquelle R₁' et R₂' sont constitués par un atome d'hydrogène ou un radical hydrocarbyl comprenant de 1 à 30 atomes de carbone, R₁ et R₂ sont des radicaux hydrocarbyl comprenant de 1 à 30 atomes de carbone,
- avec au moins un composé d'aluminium de formule AlR"ₙX₃₋ₙ dans laquelle R" est un radical hydrocarbyl comprenant de 1 à 6 atomes de carbone, X est un atome de chlore ou de brome, et n est un nombre compris entre 1 et 2,
- et avec au moins un composé d'aluminium de formule AlR'''₃ dans laquelle R''' est un radical hydrocarbyl comprenant de 1 à 6 atomes de carbone.

2. Composition catalytique selon la revendication 1, **caractérisée en ce qu'**elle contient également au moins un solvant choisi dans le groupe formé par les hydrocarbures aliphatiques, cycloaliphatiques et aromatiques, et les sous-produits d'oligomérisation tels que les oligomères supérieurs.

3. Composition catalytique selon l'une des revendications 1 à 2, **caractérisée en ce que** le solvant est choisi dans le groupe formé par les hydrocarbures aromatiques.

4. Composition catalytique selon l'une des revendications 1 à 3, **caractérisée en ce que** le solvant est l'ortho-xylène.

5. Composition catalytique selon la revendication 1, **caractérisée en ce que** le composé de zirconium et le composé organique sont mélangés, le produit obtenu étant ensuite mélangé avec les composés d'aluminium.

6. Composition catalytique selon l'une des revendications 1 à 5, **caractérisée en ce que** le composé organique est choisi dans le groupe formé par le diéthoxyméthane, le diisopropoxyméthane, le diéthoxy-1,1-éthane, le diisobutoxy-1,1-éthane, le diméthoxy-1,1-décane, le nonyl-2-dioxolane-1,3, le diméthoxy-2,2-octane, le diméthoxy-1,1-cyclohexane, le diméthoxy-2,2-propane, le dibutoxy-2,2-propane, le dihexyloxy-2,2-propane, le dioctoxy-2,2-propane, le di-(éthyl-2-hexyloxy)-2,2-propane.

7. Composition catalytique selon l'une des revendications 1 à 6, **caractérisée en ce que** le composé de zirconium est le tétrachlorure de zirconium.

8. Composition catalytique selon l'une des revendications 1 à 7, **caractérisée en ce que** les deux composés d'aluminium sont le sesquichlorure d'éthylaluminium et le triéthylaluminium.

9. Composition catalytique selon l'une des revendications 1 à 8, **caractérisée en ce que** le rapport molaire entre le composé organique et le composé de zirconium est compris entre 0,1:1 et 5:1.

10. Composition catalytique selon l'une des revendications 1 à 9, **caractérisée en ce que** le rapport molaire entre le composé chloré ou bromé d'aluminium hydrocarbyl et le composé de zirconium est compris entre 1:1 et 100:1.

11. Composition catalytique selon l'une des revendications 1 à 10, **caractérisée en ce que** le rapport molaire entre le composé de tris(hydrocarbyl)-aluminium et le composé de zirconium est compris entre 0,01:1 et 10:1.

12. Composition catalytique selon l'une des revendications 1 à 11, **caractérisée en ce que** le mélange des composants du catalyseur s'effectue à une température comprise entre -10 et 180 °C.

13. Composition catalytique selon l'une des revendications 2 à 4, **caractérisée en ce que** le solvant est utilisé lors du mélange des composés.

14. Procédé pour la conversion d'éthylène en oléfines alpha légères avec une composition catalytique selon l'une des revendications 1 à 13.

## Patentansprüche

1. Katalytische Zusammensetzung, **dadurch gekennzeichnet, dass** die Zusammensetzung erhalten wird durch Mischung:
- von wenigstens einer Zirkoniumverbindung der Formel ZrXₓY_{y}O_{z}, in der X ein Chlor- oder Bromatom ist, Y ein Rest ist, der aus der durch Alkoxyreste RO⁻, Amidoreste R₂N⁻, Carboxylatreste RCOO⁻ gebildeten Gruppe gewählt ist, wobei R ein Kohlenwasserstoffrest ist, der 1 bis 30 Kohlenstoffatome umfasst, x und y ganze Werte zwischen 0 und 4 annehmen können und z gleich 0 oder 0,5 ist, wobei die Summe x+y+2z gleich 4 ist,
- mit wenigstens einer organischen Verbindung einer Fromel (R₁')(R₂')C(OR₁)(OR₂), in der R₁' und R₂' aus einem Wasserstoffatom oder einem Kohlenwasserstoffrest, der 1 bis 30 Kohlenstoffatome umfasst, bestehen und R₁ und R₂ Kohlenwasserstoffreste sind, die 1 bis 30 Kohlenstoffatome umfassen,
- mit wenigstens einer Aluminiumverbindung einer Fromel AlR"ₙX₃₋ₙ, in der R" ein Kohlenwasserstoffrest ist, der 1 bis 6 Kohlenstoffatome umfasst, X ein Chlor- oder Bromatom ist und n eine ganze Zahl zwischen 1 und 2 ist,
- und mit wenigstens einer Aluminiumverbindung einer Fromel AlR'''₃, in der R"' ein Kohlenwasserstoffrest ist, der 1 bis 6 Kohlenstoffatome umfasst.

2. Katalytische Zusammensetzung nach Anspruch 1, **dadurch gekennzeichnet, dass** sie auch wenigstens ein Lösungsmittel enthält, das aus der Gruppe gewählt ist, die gebildet wird durch die aliphatischen, zycloaliphatischen und aromatischen Kohlenwasserstoffe und die Oligomerisierungsnebenprodukten wie höheren Oligomeren.

3. Katalytische Zusammensetzung nach einem der Ansprüche 1 bis 2, **dadurch gekennzeichnet, dass** das Lösungsmittel aus der Gruppe gewählt ist, die gebildet wird durch die aromatischen Kohlenwasserstoffe.

4. Katalytische Zusammensetzung nach einem der Ansprüche 1 bis 3, **dadurch gekennzeichnet, dass** das Lösungsmittel ortho-Xylol ist.

5. Katalytische Zusammensetzung nach Anspruch 1, **dadurch gekennzeichnet, dass** die Zirkoniumverbindung und die organische Verbindung gemischt werden, wobei das erhaltene Produkt anschließend mit den Aluminiumverbindungen vermischt wird.

6. Katalytische Zusammensetzung nach einem der Ansprüche 1 bis 5, **dadurch gekennzeichnet, dass** die organische Verbindung aus der Gruppe gewählt ist, die gebildet wird durch Diethoxymethan, Diisopropoxymethan, 1,1-Diethoxyethan, 1,1,-Diisobutoxyethan, 1,1-Dimethoxydecan, 2-Nonyl-1,3-Dioxolan, 2,2-Dimethoxyoctan, 1,1-Dimethoxyzyclohexan, 2,2-Dimethoxypropan, 2,2-Dibutoxypropan, 2,2-Dihexyloxypropan, 2,2-Dioctoxypropan, 2,2-Di-(2-Ethylhexloxy)-Propan.

7. Katalytische Zusammensetzung nach einem der Ansprüche 1 bis 6, **dadurch gekennzeichnet, dass** die Zirkoniumverbindung Zirkoniumtetrachlorid ist.

8. Katalytische Zusammensetzung nach einem der Ansprüche 1 bis 7, **dadurch gekennzeichnet, dass** die beiden Aluminiumverbindungen das Ethylaluminiumsesquichlorid und Triethylaluminium sind.

9. Katalytische Zusammensetzung nach einem der Ansprüche 1 bis 8, **dadurch gekennzeichnet, dass** das molare Verhältnis zwischen der organischen Verbindung und der Zirkoniumverbindung zwischen 0,1:1 und 5:1 liegt.

10. Katalytische Zusammensetzung nach einem der Ansprüche 1 bis 9, **dadurch gekennzeichnet, dass** das molare Verhältnis zwischen der chlorierten oder bromierten Aluminiumkohlenwasserstoffverbindung und der Zirkoniumverbindung zwischen 1:1 und 100:1 liegt.

11. Katalytische Zusammensetzung nach einem der Ansprüche 1 bis 10, **dadurch gekennzeichnet, dass** das molare Verhältnis zwischen der Tris-(Kohlenwasserstoff)-Aluminiumverbindung und der Zirkoniumverbindung zwischen 0,01:1 und 10:1 liegt.

12. Katalytische Zusammensetzung nach einem der Ansprüche 1 bis 11, **dadurch gekennzeichnet, dass** das Mischen der Bestandteile des Katalysators bei einer Temperatur zwischen -10°C und 180°C geschieht.

13. Katalytische Zusammensetzung nach einem der Ansprüche 2 bis 4, **dadurch gekennzeichnet, dass** das Lösungsmittel bei der Mischung der Verbindungen verwendet wird.

14. Verfahren zur Umwandlung von Ethylen zu leichten alpha-Olefinen mit einer katalytischen Zusammensetzung nach einem der Ansprüche 1 bis 13.

## Claims

1. A catalytic composition, **characterized in that** said catalytic composition is obtained by mixing:
• at least one zirconium compound with formula ZrXₓY_{y}O_{z}, where X is a chlorine or bromine atom, Y is a radical selected from the group formed by alkoxy RO⁻, amido R₂N⁻, and carboxylate RCOO⁻ groups, where R is a hydrocarbyl radical containing 1 to 30 carbon atoms, x and y have whole number values of 0 to 4 and z is equal to 0 or 0.5, the sum x + y + 2z being equal to 4;
• with at least one organic compound with formula (R₁')(R₂')C(OR₁)(OR₂) where R₁' and R₂' are constituted by a hydrogen atom or a hydrocarbyl radical containing 1 to 30 carbon atoms, R₁ and R₂ being hydrocarbyl radicals containing 1 to 30 carbon atoms;
• with at least one aluminium compound with formula AlR"ₙX₃₋ₙ where R" is a hydrocarbyl radical containing 1 to 6 carbon atoms, X is a chlorine or bromine atom, and n is a number in the range 1 to 2;
• and with at least one aluminium compound with formula AlR'''₃ where R''' is a hydrocarbyl radical containing 1 to 6 carbon atoms.

2. A catalytic composition according to claim 1, **characterized in that** it also contains at least one solvent selected from the group formed by aliphatic, cycloaliphatic and aromatic hydrocarbons, and by-products of oligomerisation such as high oligomers.

3. A catalytic composition according to claim 1 or claim 2, **characterized in that** the solvent is selected from the group formed by aromatic hydrocarbons.

4. A catalytic composition according to any one of claims 1 to 3, **characterized in that** the solvent is ortho-xylene.

5. A catalytic composition according to claim 1, **characterized in that** the zirconium compound and the organic compound are mixed, the product obtained then being mixed with the aluminium compounds.

6. A catalytic composition according to any one of claims 1 to 5, **characterized in that** the organic compound is selected from the group formed by diethoxymethane, diisopropoxymethane, 1,1-diethoxyethane, 1,1-diisobutoxyethane, 1,1-dimethoxydecane, 2-nonyl-1,3-dioxolane, 2,2-dimethoxyoctane, 1,1-dimethoxycyclohexane, 2,2-dimethoxypropane, 2,2-dibutoxypropane, 2,2-dihexyloxypropane, 2,2-dioctoxypropane, and 2,2-di-(2-ethylhexyloxy)-propane.

7. A catalytic composition according to any one of claims 1 to 6, **characterized in that** the zirconium compound is zirconium tetrachloride.

8. A catalytic composition according to any one of claims 1 to 7, **characterized in that** the two aluminium compounds are ethylaluminium sesquichloride and triethylaluminium.

9. A catalytic composition according to any one of claims 1 to 8, **characterized in that** the molar ratio between the organic compound and the zirconium compound is in the range 0.1:1 to 5:1.

10. A catalytic composition according to any one of claims 1 to 9, **characterized in that** the molar ratio between the chlorine-containing or bromine-containing aluminium hydrocarbyl and the zirconium compound is in the range 1:1 to 100:1.

11. A catalytic composition according to any one of claims 1 to 10, **characterized in that** the molar ratio between the tris-(hydrocarbyl)-aluminium compound and the zirconium compound is in the range 0.01:1 to 10:1.

12. A catalytic composition according to any one of claims 1 to 11, **characterized in that** the catalyst components are mixed at a temperature which is in the range -10°C to 180°C.

13. A catalytic composition according to any one of claims 2 to 4, **characterized in that** the solvent is used during mixing of the compounds.

14. A process for converting ethylene to light alpha olefins using a catalytic composition according to any one of claims 1 to 13.
